# EUROPEAN PATENT APPLICATION

(11) **EP 2 902 065 A1**
(43) Date of publication of application: **05.08.2015**
(21) Application number: 14176164.3
(22) Date of filing: 08.07.2014
(51) Int. Cl.: A61M 15/08, A61M 21/00, A61L 9/12, A61M 15/00

(54) **Portable devices and methods for providing scents**

(30) Priority: 10.07.2013 US 201313938919; 07.07.2014 US 201414324881
(71) Applicant: Woodpecker Laboratories, LLC, New York, NY 10040 (US)
(72) Inventor: Rome, Zachary David, NEW YORK, NY New York NY 10040 (US)
(74) Representative: Carangelo, Pierluigi

(57) **Abstract**

The present technology is directed to devices and methods for providing scents to a user - in particular, in a portable way that can dispense a plurality of scents to an individual user based on the individual user's personal preferences or needs.

## Description

### TECHNICAL FIELD

This invention relates generally to devices and methods for providing scents to a user - in particular, in a portable way that can dispense a plurality of scents to an individual user based on the individual user's personal preferences or needs.

### BACKGROUND

The concept of aromatherapy has been the subject of studies documenting the effects of particular scents on behavior, emotions, memory, and performance. Additionally, studies have shown that different scents can elicit different psychological and biological responses, and that individuals strongly develop their own unique associations connecting scents and memories/emotions. What an individual smells has a much bigger impact on his or her day-to-day life than many may realize. Evidenced-based science has revealed just how significant the effects of scents can be on behavior, emotions, memory, and performance. For example, it is now widely accepted that smell has stronger ties to memory than any other sense. According to the Sense of Smell Institute (http://www.senseofsmellinstitute.org/mss-fun-facts.php), people report recalling smells with about 65% accuracy after a year, compared with a recall of about 50% after three months for vision.

Like one's sense of vision or hearing, one can lose one's sense of smell with age. Unlike one's vision or hearing, however, there are exercises one can do to protect and even sharpen the sense of smell. A recent article in the Wall Street Journal ("Uncork the Nose's Secret Powers" February 12, 2013) estimates that "[b]y age 60, about half of people will experience a reduction in their ability to smell, and by age 80, about three-fourths will." According to this article, it has been shown that by sniffing 3-4 different scents around 4-6 times a day, an individual can eventually spark different receptors that will help train the brain to discern differences among scents.

Different products and technologies have been designed to dispense scents to evoke different psychological and biological responses. For example, U.S. Patent Application Publication No. 2009/0123548 shows an inhaler for administering aromatherapy. This device has an aromatherapy composition housed within a container. The container has an opening where the composition can be inhaled through the mouth and/or nose. While this device provides a method for delivering an odor, only one odor is capable of being carried and/or delivered.

U.S. Patent No. 7,691,336 shows a device that is capable of dispensing multiple odors. This device uses a power supply, a heater, and a fan to sequentially emit different volatile materials. Because this device is designed to emit scents broadly into the environment, it is not well suited to provide therapeutic effects to only a single user, for example in a crowded environment. Additionally, this device is not portable; it is also less durable, more costly, and involves a great number of moving parts and maintenance due to the requirement of features such as a power source, a heater, a fan, and a safety interlock. Further, the sequential emitting of scents limits a user's ability to conveniently experience a particular aroma at the time he or she might desire.

Thus, no device formerly developed provides a means of conveniently carrying a diversity of scents that can be portably and individually dispensed by simple, solely or substantially mechanical (that is, non-electric) methods as the user desires. A need exists for devices that are easily transported and carried, and that are useful in providing multiple scents for personal use, based on an individual's desire to be exposed to certain scents.

### BRIEF SUMMARY

The present technology is directed, in certain embodiments, to a portable device for providing a scent, the device having a length comprising a first cartridge compartment connected to a second connected cartridge compartment; wherein the first cartridge compartment encloses a first cartridge, the first cartridge comprising a material having a first scent; and wherein the second compartment encloses a second cartridge, the second cartridge comprising a material having a second scent.

The present technology is directed, in other embodiments, to a portable device having a substantially elongated shape, the substantially elongated shape having a length comprising a first cartridge compartment connected to a second connected cartridge compartment; wherein the first cartridge compartment is threadably connected to and encloses a first cartridge, the first cartridge comprising a material that emits a first scent; and wherein the second compartment is threadably connected to and encloses a second cartridge, the second cartridge comprising a material that emits a second scent that is different from the first scent; the first cartridge compartment being movable to expose the first cartridge and emit the first scent; and the second cartridge compartment being movable to expose the second cartridge and emit the second scent.

The present technology is directed, in other embodiments, to a method of providing two different scents to an individual user, the method comprising the steps of:
(a) providing a portable device having a length comprising a first cartridge compartment connected to a second connected cartridge compartment, wherein the first cartridge compartment encloses a first cartridge, the first cartridge comprising a material having a first scent; and wherein the second compartment encloses a second cartridge, the second cartridge comprising a material having a second scent that is different from the first scent;
(b) moving a portion of the first cartridge compartment to expose a portion of the first cartridge, thus emitting the first scent; and
(c) moving a portion of the second cartridge compartment to expose a portion of the second cartridge, thus emitting the second scent.

The present technology is directed, in other embodiments, to a portable device for providing a scent, the device having a length comprising a first cartridge compartment connected to a second connected cartridge compartment;
wherein the first cartridge compartment comprises a proximal end and a distal end, and encloses a first reservoir comprising a liquid, gas or solid composition having a first scent; and
wherein the second cartridge compartment comprises a proximal end and a distal end, and encloses a second reservoir comprising a liquid, gas or solid having a second scent.

### BRIEF DESCRIPTION OF THE DRAWINGS

FIG. 1 shows a certain embodiment of a device according to the present technology.
FIG. 2 shows another embodiment of a device according to the present technology, with both internal and external features.
FIG. 3 shows a cartridge in accordance with another embodiment.
FIG. 4 shows a cartridge and its component parts in accordance with another embodiment.
FIG. 5 shows a close-up view of another embodiment.
FIG. 6 shows an exterior view of a device according to another embodiment of the present technology.
FIG. 7 shows another embodiment of a device according to the present technology, with both internal and external features.
FIG. 8 shows a closeup of the interior of a portion of a device in accordance with another embodiment.
FIG. 9 shows the internal and external components of a device in accordance with another embodiment.
FIG. 10 shows the components of a device in accordance with another embodiment.

### DETAILED DESCRIPTION

As used herein, any reference to any of the "first cartridge compartment" or "first cartridge" may also apply to any of the subsequent (second, third, fourth and so on) cartridge compartments and cartridges. Further, the general terms "cartridge compartment" and "cartridge," are understood to refer broadly and generally to any of the first, second, third, fourth and so on of these terms. Moreover, the present technology contemplates embodiments wherein a given cartridge comprises a cartridge compartment, and wherein the cartridge comprises nothing in its interior other than a space that can hold a composition or material (liquid, solid or gas, including vapor) that has a scent.

In certain embodiments, a device is provided herein that comprises one or more individual units (e.g., cartridge compartments) that each comprise an absorbent material saturated with a liquid comprising a volatile compound. As used herein, "volatile" is used to refer to any compound that is capable of releasing a scent, and can be impregnated into an absorbent material for the purpose of providing a scent to a user when exposed to air or the immediate environment of the material, such that a user can experience the scent.

As used herein, "portable" refers to anything that can be easily removed and carried around by an individual. For example, the present disclosure contemplates portable devices that are, in various embodiments, small enough to carry in a backpack, suitcase, pocket or handbag, that can be placed on a desk or table next to a user or that can be carried in a user's hand. However, it is important to note that a device need not be tiny to be portable. So long as the device is capable of being carried by an individual, it is portable for purposes of the embodiments herein.

In certain embodiments, a portable device may or may not be connected to a power source such as an electric source. In certain embodiments, the advantage of certain devices described herein is the ability for a user to use them without the need for electric power, *e.g*., power supplied by connection to an electrical source such as through a wall socket or a battery.

Throughout the present disclosure, reference is made to a portable device having a substantially elongated shape. As used herein, "substantially elongated shape" means a shape that has a longer length than width.

As used herein, "scent" means something that can be detected by a user's sense of smell, and includes any fragrance or odor, whether pleasant or unpleasant.

In various embodiments of the present technology, the devices and methods herein are useful for application to an individual user - that is, only one user and no more. In other embodiments, the devices and methods may be applicable for a plurality (that is, two or more) of users.

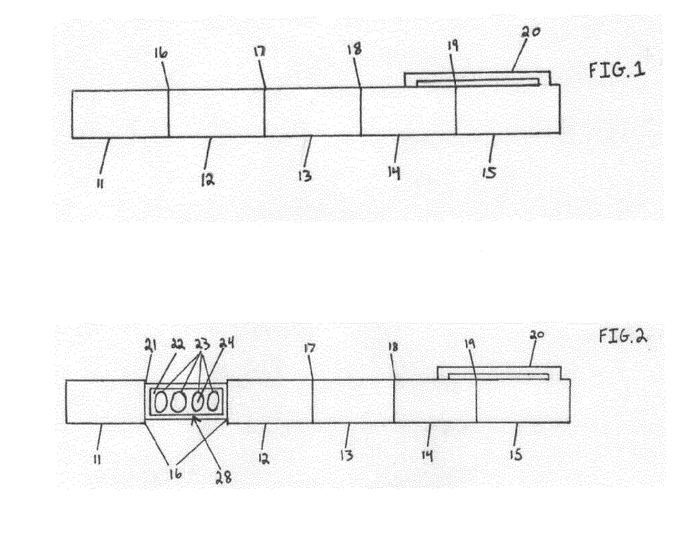

In various embodiments, a device according to the present technology comprises two or more cartridge compartments that are connected, *e.g*., end to end. These cartridge compartments are the basic segments that together make up the length of the body of the device. For example, as shows in FIGS. 1 and 2, a device in accordance with certain embodiments herein may comprise a series of cartridge compartments connected end to end, and any one or more (or each) of these cartridge compartments may be removably attached to any cartridge compartment immediately adjacent to it. FIG. 2 also shows the partial interior of a device according to certain embodiments, wherein at least one cartridge compartment is attached to the cartridge compartment immediately adjacent to it via a threaded connection (throughout the present disclosure, also referred to as "threadably" attached).

In certain embodiments, it can be seen that in operation an individual user may use the device as a vehicle for dispensing different pre-selected scents as the user may desire. As can be seen in FIGS. 1 and 2, in certain embodiments, a device in accordance with the present technology comprises a first cartridge compartment 11, a second cartridge compartment 12 and so forth (13, 14 and 15 being the third, fourth and fifth cartridge compartments respectively, although the embodiments herein may have any number of cartridge compartments according to the preference of the user or manufacturer). In the embodiment shown in FIGS. 1 and 2, each of the cartridge compartments is connected to the one immediately adjacent to it via a connection point. That is, the first cartridge compartment 11 and the second cartridge compartment 12 are connected via a connection point 16. The second cartridge compartment 12 and the third cartridge compartment 13 are connected via a connection point 17. The third cartridge compartment 13 and the fourth cartridge compartment 14 are connected via a connection point 18, and so forth. Any cartridge compartment may be connected to the cartridge compartment immediately adjacent to it via any connection mechanism, including but not limited to: a threaded connection, a snap closure, a binary connection (such as one that can be attached or detached by uniting complementary parts and then twisting them to engage the parts and secure the connection), a spring mechanism, a magnetic connection, a loop and hook connection, an adhesive or the like.

In the embodiment shown in FIG. 2, the cartridge 28 (which is enclosed either partially or completely by the cartridge compartment 11) comprises a wick 24 containing (for example, saturated or impregnated with) a composition comprising a scent. In certain embodiments, the scent may be present in the form of a liquid such as an essential oil. The wick 24 may be contained in whole or in part within the cartridge's casing 22 and may serve as the source of the scent that is diffused through the one or more cartridge vents 23 into the surrounding environment.

Thus, in use, the user can move a portion or all of the first cartridge compartment 11 (or any of the other cartridge compartments 12, 13, 14, and 15 shown in FIGS. 1 and 2) to expose any or all of the cartridge 28. In certain embodiments where any of the cartridge compartments is removably attached to the cartridge compartment immediately adjacent to it via a connection point, the cartridge compartment that is desired to be moved in whole or in part can be either unscrewed, unsnapped, pulled apart by the force of the user's hand or slid back as the case may be, for any distance sufficient to expose any portion of the cartridge and therefore permit release of the scent into the immediate vicinity of the device.

Once the cartridge is exposed, the user can choose to experience a concentrated dose of the scent by placing the cartridge close to his or her nose and breathing in the odor, or even inserting a portion of the cartridge directly into a nostril. Alternatively, the user can choose to experience a less concentrated dose of the scent by, for example, having the cartridge exposed in his or her immediate vicinity (for example, holding the exposed section of the cartridge up near his face, hanging it from his neck or clipping it to his clothing), or exposing only a portion of the cartridge.

In certain embodiments, the user may opt to remove the first cartridge compartment (or any of the cartridge compartments) completely from the remainder of the device. In various embodiments, the removal of the cartridge compartment will fully expose the cartridge. However, in other embodiments the user may opt to move the first cartridge compartment (or any of the cartridge compartments) a distance that exposes only part of the cartridge. As an example, in certain embodiments, the vents 23 may be disposed on the cartridge in such a manner so as to maximize the exposure of the scent while minimizing the required movement of the cartridge compartment. For example, in one embodiment they could be substantially present toward one end of the cartridge such that exposing only a small portion of the cartridge will unleash a large amount of scent, while in another embodiment they could be substantially evenly distributed along the length of the cartridge so as to provide a more measured release of scent, while in yet another they could be substantially present toward one end of the cartridge such that removing the entire cartridge compartment and holding the exposed end of the cartridge near or in the user's nostril delivers a concentrated burst of scent to the user.

In certain embodiments, the cartridge compartment may be prevented from complete removal by the presence of a cartridge compartment lip 21, which catches the cartridge compartment. In certain embodiments, the cartridge compartment lip 21 allows for the cartridge compartments to be separated from each other and opened without being unintentionally removed from the base device. In other embodiments, the lip 21 may either not be present, or may be removable or flexible in such a manner that the user can easily detach the first cartridge cover 11 from the second cartridge cover (or any cartridge cover from the one immediately adjacent to it) in a manner that exposes the cartridge within it, allowing the scent to escape the device into the environment.

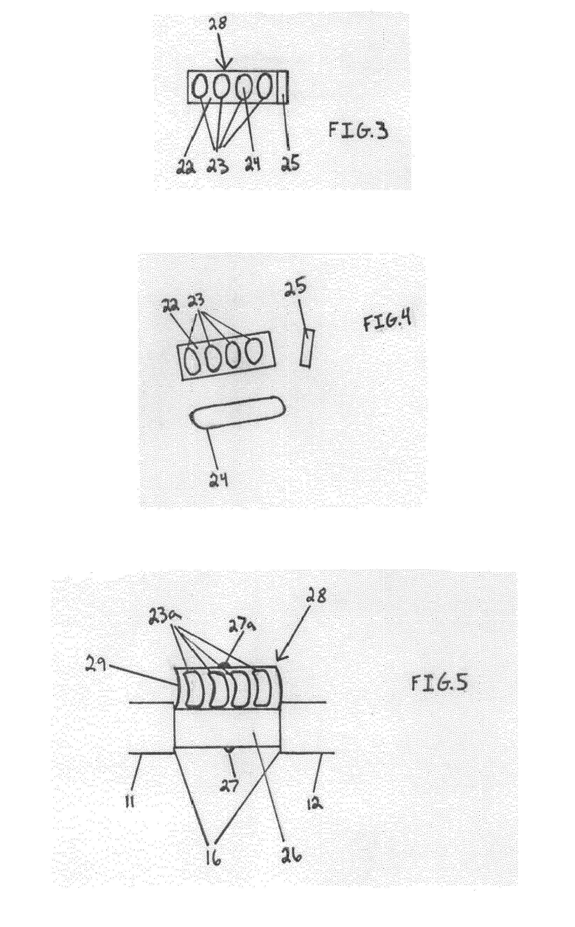

In various embodiments, the absorbent material may be disposable or may have the ability to be used more than once - for example, may either need to be removed and replaced after a certain number of uses, or may be capable of being re-impregnated multiple times with the same or different scented composition(s). As can be seen in FIG. 3 and FIG. 4, in certain embodiments the cartridge 28 may comprise a cartridge casing 22 containing one or more vents 23 (also referred to herein as "vent holes"), and encloses the wick 24. In certain embodiments, the cartridge cap 25 may be removed to access the wick, for example to remove or replace it. Cartridges 28 (as shown in FIGS. 2, 3, and 4) can be acquired by the user pre-scented, or the user can obtain a blank cartridge and use their own stock of scents to provide it with a scent. Among other possibilities, a user can easily do this by removing the cartridge cap 25, removing the wick 24 and saturating it in an essential oil. The wick can then be returned to the cartridge casing 22 and inserted into the device.

When the user wishes to cease the diffusion of the scent, it is only necessary to seal the cartridge compartment by reversing the movements that led to exposure of the cartridge - e.g., screwing it closed, snapping the two adjacent cartridge compartments back together, releasing the spring that has forced the two adjacent cartridge compartments apart, or otherwise reconnecting the two cartridge compartments at the respective connection 16, 17, 18, and/or 19. In certain embodiments, each of the connections 16, 17, 18, and 19, when closed, creates an airtight or near-airtight seal, to prevent odors from escaping or entering the cartridge compartment.

In certain embodiments, a clip 20 extends from the topmost cartridge compartment 15, or any of the cartridge compartments, to enhance carrying convenience - for example, in the user's pocket or attached to a belt or strap carried or worn by the user.

FIG. 5 shows another embodiment in accordance with the present technology. In this embodiment shown, the cartridge 28 is not necessarily removable from the device. It may comprise instead a receptacle 26 that is designed to accept a material capable of being impregnated with the scent saturated in a composition containing the scent. Once this material is in place, a cartridge lid 29 that is, in various embodiments, hingedly attached to the cartridge, can be closed by connecting the two ends of the snap closure 27 and 27a. The scent from the material is diffused through the one or more cartridge vents 23a and the user can then choose to experience the scent in the same manner as previously discussed.

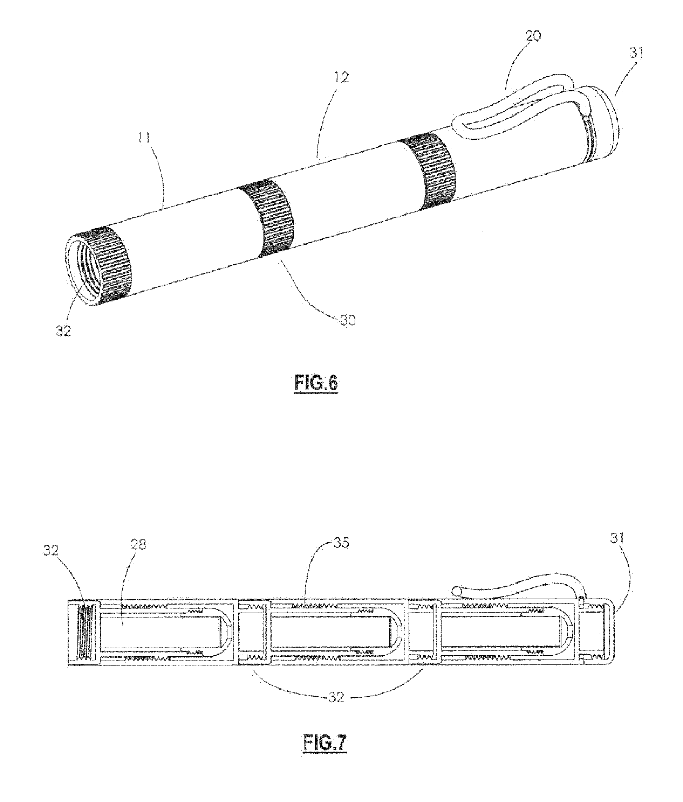

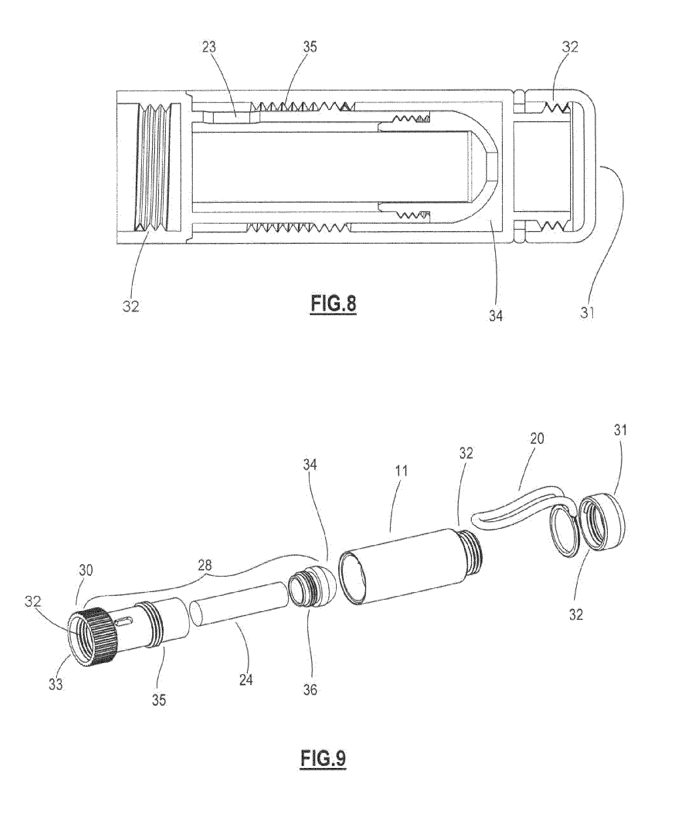

FIG. 6 shows another embodiment in accordance with the present technology. As shown therein, the device is in the form of a "pen"-like structure that the user can carry around easily in a pocket or handbag, or clipped to a belt, strap or anything else on or near the user's body. In the embodiments shown here, the cartridge is longer than the cartridge compartment that encloses it, and the extra length portion comprises a collar 30 that extends beyond the length of the cartridge compartment 11 and provides a "stop" for the cartridge compartment when the cartridge 28 is fully inserted into the cartridge compartment 11. In certain embodiments, the collar 30 may be of a different material than the exterior of one or more of the cartridge compartments, or may be of the same material but having a different texture, such that a user can grip the collar to more easily separate adjacent cartridge compartments and access the scent within. In various embodiments, the collar 30 can be a separate component that is attached to the cartridge 28, or it can be a partially or fully integrated part of the cartridge (for example, as shown in FIG. 9). As can be seen in FIG. 6, the first cartridge compartment 11 is connected to the second cartridge compartment 12, via connection with the collar 30.

FIG. 7 shows an interior view of three adjacent cartridge compartments of a device in accordance with the present technology. As can be seen therein, each of the three cartridge compartments are connected via end threads 32. Referring to the device in FIG. 7 as having a "proximal" end (to the left) and a "distal" end (to the right, in the direction of the clip 20), in certain embodiments, end threads 32 may be present in any of the following: the proximal end of the collar 30, or the distal end of an adjacent cartridge compartment (permitting it to be threadably attached to an adjacent cartridge compartment. In certain embodiments, at the distal-most of the cartridge compartments, the end threads 32 will mate with complementary end threads 32 on an end piece 31 (seen more clearly in FIGS. 7 and 8).

Further, in certain embodiments, a cartridge compartment 11 will comprise a cartridge 28 having internal threads 35 disposed at some point along its length. As can be seen in FIGS. 7 through 9, when the cartridge is fully inserted into the cartridge compartment, these internal threads 35 mate with complementary internal threads that are on the interior of the cartridge compartment, to secure the connection.

FIG. 8 shows a close-up of an interior view of a single cartridge compartment of a device according to the present technology, and specifically, a threading connection. As can be seen in FIG. 8, in certain embodiments a vent hole 23 can still emit scent into the interior of the cartridge compartment even when the cartridge compartment is fully closed. This can hasten release of the scent as soon as the user opens the cartridge compartment even a small amount.

FIG. 9 shows certain components in detail of an embodiment of the present technology. In this embodiment, only a first cartridge compartment is present (herein it will be referred to as merely the "cartridge compartment"), although as discussed throughout the present disclosure, the present technology contemplates any number of cartridge compartments. As can be seen herein, the cartridge compartment 11 is a hollow tube into which the cartridge 28 and nose piece 34 are inserted, the wick 24 being disposed therein. In the embodiment shown here, the device also includes the clip 20 and the end piece 31. The bottom 33 of the cartridge 28 on the exposed proximal end of the collar portion 30 comprises end threads 32 that are optional for the first compartment. In certain embodiments, for a second or subsequent compartment, these end threads 32 are configured to line up with and correspond to end threads 32 on the distal end of the an adjacent cartridge compartment.

In certain embodiments, the cartridge comprises two pieces that can be separated to access the space within, and when held together enclose the material (for example, the wick) having the scent. In such embodiments, the cartridge may comprise a separate nose piece 34, as shown, for example, in FIG. 9. The nose piece 34 may comprise nose threads 36 that line up with and correspond to nose threads on the remainder of the cartridge (the nose threads in the remainder of the cartridge are not visible in FIG. 9 because in that embodiment, they are on the inside of the cartridge). As used herein, the "remainder of the cartridge" means the portion of the cartridge that is separate from the nose piece, and that connects with the nose piece to enclose the material having the scent. The nose piece 34 may be engaged with the remainder of the cartridge to enclose the wick 24. The entire cartridge 28 is then slid into the cartridge compartment 11, and the clip 20 and end piece 31 are attached to provide the convenient portable device.

In another embodiment, not shown in the Figures, the device as shown in FIG. 9 can comprise a second, third or subsequent cartridge compartment(s). In such embodiments, the end threads 32 at the distal end of the cartridge 28 would, rather than corresponding to and complementing threads 32 on the end piece 31, correspond to and complement threads on the bottom 33 of the collar portion 32 of the next cartridge. In such an embodiment, the clip 20 could be present only on one end of the device, for the user's convenience. However, a device could include multiple clips 20, based on the user's preference.

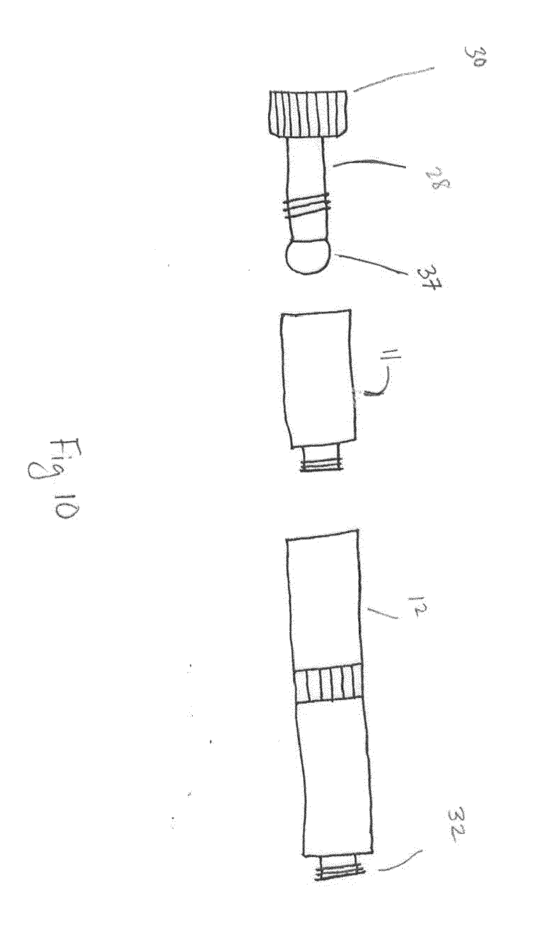

In other embodiments, one or more of the cartridges or cartridge compartments comprises a ball 37 in contact with one of its ends. In a non-limiting example, as shown in FIG. 10, a device according to the present technology comprises a cartridge 28 that, rather than having vents 23, is closed along its length and comprises a ball 37 in contact with one end, its distal end or its proximal end. In such an embodiment, the composition comprising the scent can be disposed within the interior of the cartridge 28, and may comprise a liquid, gas (*e.g*., vapor) or solid. The ball 37 may be attached to the distal end of the cartridge 28 such that it can rotate, contacting the composition comprising the scent (whether in liquid, gas or solid form) within the cartridge, thus permitting the user to transfer the composition from the interior of the cartridge to the outside, and then touching the ball (now covered in part or whole with the composition comprising the scent) with any of a number of items, including the user's body, or the ambient air, as, for example, a "roll-on" device.

In certain embodiments, the cartridge 28 may comprise both a ball 37 and one or more vents 23, to maximize the diffusion of the scent outward, permitting the user to both smell the scent and also roll the scent onto his or her body through different parts of the cartridge.

In various embodiments, a device according to the present technology may comprise any of the first, second or third (or any subsequent) cartridge compartments having any configuration of vents and balls, as described herein. In certain embodiments, the ball may be present along with, or incorporated with, the nose piece 34 as previously described herein; for example, a nose piece 34 may be included as a cover for the ball to keep the scent contained when not in use.

The present technology contemplates further applications. For example, in various embodiments, the devices herein may further comprise additional features such as a flashlight, a pen, a keychain, a carabiner attachment or the like. Thus, while in some embodiments the devices herein do not include any power source, in other embodiments they may include power sources such as, e.g., batteries or conduits to electrical sources such as an electrical outlet, to power any of the additional features.

The devices discussed herein may be made in whole or part from any material that is light, easy to use and relatively inexpensive, including but not limited to polymeric materials, including injection molded plastics and plastic blends, e.g., acylonotrile-butadiene-styrene ABS, nylon, polycarbonate, polystyrene, polyethylene, polypropylene and the like; as well as metal, *e.g*., aluminum or steel; rubber, silicone or the like. In certain embodiments, the devices may be made through injection molding, or through 3-dimensional printing.

In certain embodiments, the present technology also contemplates methods for improving a user's sense of smell, by, *e.g*., permitting a user to experience a plurality of scents as a way to "train" his or her nose to recognize such scents. The olfactory training aspect of the present technology is of great significance. Research shows that doing such "training" not only improves a person's ability to recognize scents, but also improves their sensitivity to scents and ability to discriminate between them.

One's ability to smell generally has three measures: sensitivity, discrimination and identification. Thus, in certain embodiments, any one or more of these may be improved in accordance with the present technology. In certain embodiments, the present technology contemplates methods for improving a user's sense of smell, *e.g*., permitting a user to experience a plurality of scents as a way to "train" his or her nose to recognize or identify such scents, or to discriminate between them or improve their sensitivity to them. For example, a user could load two or more known scents into a device in accordance with the embodiments herein, and practice smelling such scents, thus permitting his or her olfactory senses to become trained to the scents and becoming able to discern or identify the scents after training in a way that he or she was unable to discern before the training. As a result, in certain embodiments a user's sense of smell could be improved in that the methods discussed herein would result in an improvement of the user's ability to discern the two scents; or, for example, by the user's reporting an increased accuracy or increased rate of positive identification of one or more of the scents when exposed to a scent of unknown identity. In another embodiment, the user could be trained to discern a scent at a lower concentration than before the training - this could, for example, be shown by first testing the lowest amount of a particular scent that a user can discern, then training the user in accordance with the methods discussed herein, and then showing that the user can discern the scent at a lower concentration than he or she could before the training. In yet another embodiment, a user could be trained to distinguish or tell the difference between two scents that might smell similar; after training the user could be shown to be able to tell the difference between the two scents in a way that he or she was unable to do before the training. Thus, in certain embodiments, exposing the user to the first and second scent results in the improvement of any one or more of the following: (a) the user's ability to distinguish or discern a scent; (b) the user's sensitivity to a scent; or (c)the user's ability to identify a scent of unknown identity.

Although the present technology has been described in relation to particular embodiments thereof, these embodiments and examples are merely exemplary and not intended to be limiting. Many other variations and modifications and other uses will become apparent to those skilled in the art. The present technology should, therefore, not be limited by the specific disclosure herein, and may be embodied in other forms not explicitly described here, without departing from the spirit thereof.

## Claims

1. A portable device for providing a scent, the device having a length comprising a first cartridge compartment connected to a second connected cartridge compartment;
wherein the first cartridge compartment encloses a first cartridge, the first cartridge comprising a material having a first scent; and
wherein the second compartment encloses a second cartridge, the second cartridge comprising a material having a second scent.

2. The portable device of claim 1, wherein the second scent is different from the first scent.

3. The portable device of claim 1, wherein the first cartridge comprises a vent through which the first scent is emitted from the material.

4. The portable device of claim 3, wherein a portion or all of the first cartridge compartment is movable to expose a portion or all of the first cartridge such that the first scent is emitted from the material through the vent into the environment surrounding the portable device.

5. The portable device of claim 1, wherein the first cartridge comprises a ball in contact with one end of the first cartridge, wherein the ball can rotate and contact the material having the first scent.

6. The portable device of claim 1, wherein the first cartridge compartment and the first cartridge are connected to each other with a connection chosen from a threaded portion, a snap connection, a binary connection, a spring or a hinge.

7. The portable device of claim 1, wherein the first cartridge compartment and the second cartridge compartment are connected to each other with a connection chosen from a threaded portion, a snap connection, a binary connection, a spring or a hinge.

8. The portable device of claim 1, wherein the material in the first cartridge comprises a wick impregnated with a liquid, gas or solid comprising the first scent.

9. The portable device of claim 1, wherein one or more of the first cartridge or second cartridge comprises a proximal end and a distal end, and a ball in contact with the distal end.

10. A portable device having a substantially elongated shape, the substantially elongated shape having a length comprising a first cartridge compartment connected to a second connected cartridge compartment;
wherein the first cartridge compartment is threadably connected to and encloses a first cartridge, the first cartridge comprising a material that emits a first scent; and
wherein the second compartment is threadably connected to and encloses a second cartridge, the second cartridge comprising a material that emits a second scent that is different from the first scent;
the first cartridge compartment being movable to expose the first cartridge and emit the first scent; and the second cartridge compartment being movable to expose the second cartridge and emit the second scent.

11. A method of providing two different scents to an individual user, the method comprising the steps of:
(a) providing a portable device having a length comprising a first cartridge compartment connected to a second connected cartridge compartment, wherein the first cartridge compartment encloses a first cartridge, the first cartridge comprising a material having a first scent; and wherein the second compartment encloses a second cartridge, the second cartridge comprising a material having a second scent that is different from the first scent;
(b) moving a portion of the first cartridge compartment to expose a portion of the first cartridge, thus emitting the first scent; and
(c) moving a portion of the second cartridge compartment to expose a portion of the second cartridge, thus emitting the second scent.

12. The method of claim 11, wherein one of the first cartridge or second cartridge comprises a hinged portion that can be opened to expose the material.

13. The method of claim 11, wherein the first cartridge compartment is threadably connected to the first cartridge.

14. The method of claim 11, wherein the second cartridge compartment is threadably connected to the second cartridge.

15. The method of claim 11, wherein the first cartridge compartment and the second cartridge compartment are connected to each other with a threaded connection.

16. A method of improving a user's sense of smell, the method comprising the steps of providing two different scents to an individual user according to claim 11.

17. A portable device for providing a scent, the device having a length comprising a first cartridge compartment connected to a second connected cartridge compartment;
wherein the first cartridge compartment comprises a proximal end and a distal end, and encloses a first reservoir comprising a liquid, gas or solid composition having a first scent; and
wherein the second cartridge compartment comprises a proximal end and a distal end, and encloses a second reservoir comprising a liquid, gas or solid having a second scent.

18. The portable device of claim 17, wherein one or more of the first or second cartridge component comprises a ball in contact with its distal end, such that the ball can rotate, contacting the composition comprising the scent within the cartridge, thus permitting the user to transfer the composition from the interior of the cartridge to the outside.

19. A method of improving a user's sense of smell, the method comprising the method of claim 11.

20. The method of claim 19, wherein exposing the user to the first and second scent results in the improvement of any one or more of the following:
(a) the user's ability to distinguish or discern a scent;
(b) the user's sensitivity to a scent; or
(c) the user's ability to identify a scent of unknown identity.
